# EUROPEAN PATENT SPECIFICATION

(11) **EP 1 825 824 B1**
(45) Date of publication and mention of the grant of the patent: **04.11.2009**
(21) Application number: 06003797.5
(22) Date of filing: 24.02.2006
(51) Int. Cl.: A61B 17/22, A61M 25/10, A61B 17/00, A61M 37/00, A61F 2/06

(54) **Minimally invasive intravascular treatment device**
Minimalinvasive intravaskuläre Behandlungsvorrichtung
Dispositif de traitement intravasculaire à invasion minimale

(43) Date of publication of application: 29.08.2007
(73) Proprietor: National University of Ireland, Galway, Galway (IE)
(72) Inventor: Murphy, Bruce Philip, Lower Salthill Galway (IE); Lawlor, Vincent Patrick, County Offaly (IE)
(74) Representative: Lane, Cathal Michael

(56) References cited:
- EP-A1- 0 697 226
- WO-A-94/23787
- WO-A-98/22044
- WO-A-2004/096097
- US-A- 5 336 178
- US-A- 5 616 149
- US-A- 6 018 857
- US-A- 6 051 001
- US-A1- 2004 044 308
- US-A1- 2004 133 223

## Description

### Field of the Invention

The present invention relates to medical devices. In particular, the invention relates to a catheter based medical device for the treatment of internal body cavities such as arteries/veins or other hollow organs.

### Background to the Invention

Diseases of the circulatory system are the leading cause of death in the world, and the prevalence of the disease in younger patients is increasing. In addition, global society is following a trend whereby populations are exposing themselves to a greater extent to more of the risk factors associated with vascular disease.

For example, the rate of increase in obesity in Irish society was brought to public attention in a recent front-page Irish national newspaper article, "Tipping the scales: Child obesity levels triple" (The Irish Examiner 22/11/04). Furthermore, the statistics for cause of death present the true magnitude of the problem, in Ireland during the period 1998-2003 (inclusive) 40% of deaths within the state were caused by diseases of the circulatory system (source: Irish Central Statistics Office). This trend is not only a national problem, it is echoed internationally in 1998 in the United States 39% of all deaths were caused by diseases of the circulatory system (National Vital Statistics Reports 2000, Vol. 48, No. 11, July 24).

Atherosclerosis (vascular disease) is the accumulation of plaque within an artery wall. When the disease is at an advanced stage blood flow to organs, such as the heart, is reduced and as a consequence a heart attack or other acute event may occur. Balloon angioplasty was developed to reopen atherosclerotic arteries. This procedure involves inflating a miniature balloon at the site of an arterial blockage. Expansion of the balloon compresses the plaque and stretches the artery wall, this reopens the artery to its original diameter and restores blood flow (balloon angioplasty can be used on its own or as an adjunctive therapy to stenting). Angioplasty balloons are inflated to high pressures, up to 24atm (equivalent to 350 p.s.i. (2.4 x 10⁶ Pa) which is over 10 times the inflation pressure in an average car tyre). At these high pressures severe damage to the artery wall is caused. In a number of cases high pressure balloon angioplasty cannot dilate the blockage in the artery, specialist devices are then required to dilate the lesion, or bypass surgery is carried out.

This lead to the development of cutting balloons such as that disclosed in US Patent No. 5,196,024. This patent discloses a device and method for dilation or recanalisation of a diseased vessel by use of a balloon catheter with cutting edges to make longitudinal cuts in the vessel wall.

Since this first patent was filed, there has been considerable activity in the development of improved cutting balloons, with the emphasis on improving the blade-shielding capabilities of the cutting balloon.

In the balloon catheter disclosed in the aforementioned US Patent No. 5,196,024, the folds of the balloon in its collapsed state are used to shield the blades from the vessel wall during insertion and removal of the balloon catheter. One disadvantage of this arrangement is that the blades are not protected from damaging the balloon itself.

US Patent application number 2005/0137617 discloses a cutting balloon which aims to overcome this disadvantage. An elastically distensible folding member is disclosed which can be formed with a wall that is substantially shaped as a tube when the folding member is in a relaxed (i.e. unstressed) state. The tubular shaped folding member defines a tube axis and can have an axially aligned slit that extends through the wall. The folding member can be used to cover an incising element that is attached to the balloon and positioned in the lumen of the tubular folding member. During balloon inflation, the folding member can be deformed to expose the tip of the incising element to allow for a tissue incision.

US Patent Application No. 2005/0119678 of O'Brien et al. discloses an alternative solution wherein compressible sheaths made of a relatively low durometer, flexible material are mounted on the balloon to protect the operative cutting surface of a respective incising element during assembly of the cutting balloon and transit of the cutting balloon to the treatment site. Each sheath extends farther from the longitudinal axis than the corresponding incising element and makes first contact with the tissue during a balloon inflation. Once contact has been established between the tissue and the sheath, further balloon inflation causes the sheath to radially compress between the tissue and the inflatable balloon exposing the operative cutting surface for tissue incision.

US Patent Application No. 2004/0133223 of Weber also discloses the use of a resilient material which extends over the cutting edge of a blade on a cutting balloon, the resilient material deforming under compression to allow the cutting edge to pierce through.

The aforementioned US Patent No. 5,196,024, also discloses the use of a protective sheath which covers the entire balloon. Continuity of the sheath is interrupted by longitudinal grooves which serve to accommodate, guide and protect the tips of the (balloon's) cutting edges. The protective sheath prevents vessel injuries during delivery and holds the cutting edges in proper position prior to balloon inflation. As the balloon is inflated, the grooves of the protective sheath open up allowing the cutting edges to penetrate into the vessel wall producing cuts with sharp margins. After deflation, the cutting edges retract behind the protective sheath thereby avoiding injury to the vessel during withdrawal of the cutting balloon. An alternative solution to the problem of exposed blades damaging the balloon is disclosed in one embodiment in US Patent No. 5,196,024 wherein the blades are repositioned onto a plastic casing surrounding the balloon. Continuity of the casing is interrupted by longitudinal slots which increase in size as the balloon is inflated.

A similar arrangement is disclosed in US Patent No. 5,797,935, wherein a balloon activated force concentrator for use in cooperation with an inflatable angioplasty balloon includes at least one elongated flexible panel, an elongated cutting blade mounted on the outside surface of the elongated flexible panel, and an elastic circular band attached to each end of the elongated flexible panel for securing the elongated flexible panel to an angioplasty balloon.

Cutting balloons such as those discussed above are now commonly used on highly calcified lesions or stubborn lesions, sometimes on their own or prior to stent placement. However, these devices have been found to be prone to failure, are relatively large and difficult to manoeuvre within the vasculature, and are often restrictively expensive.

One of the greatest problems is associated with the removal of the cutting balloon after inflation. The pressure of the balloon can in some cases cause the cutting edges or blades to penetrate deeply into the vessel wall. To subsequently withdraw the blades from the vessel wall can require a strong force. In each of the above examples of cutting balloons, it is the balloon itself upon deflation which provides this retraction force. It has been known for difficulties in retracting the blades to occur, and in extreme cases removal of the cutting balloon has been impossible, resulting in a cutting balloon being left in a patient's coronary artery.

What is required therefore is an alternative to existing cutting balloons that will be more efficient, easier to use and safer.

As discussed above, cutting balloons are used to reopen blocked vessels, typically resulting from vascular disease. However, cutting balloons do not address the treatment of such vascular disease. With a continuing trend of people dying from vascular disease, and young patients increasingly exposing themselves to obesity together with the associated increased risk of diabetes, innovative effective therapies must be conceptualised to treat both the younger and the traditional older sufferer of vascular disease. These trends, along with technological advances, have resulted in an annual growth rate of approximately 20% in transcatheter technologies.

One of the main drivers of this growth rate is coronary drug eluting stents; however there are a number of areas where these stents cannot be used effectively; namely, chronic total occlusions, peripheral artery disease, and vulnerable plaque. Furthermore new devices and treatments are needed to treat restenosis associated with the edge of drug eluting stents and in-stent restenosis associated with bare metal stents. All of the above mentioned areas represent significant unmet clinical needs as no technology can adequately treat these conditions.

Advances in local drug delivery have proven extremely effective in the coronary arena, whereby drug-eluting stents have made a significant breakthrough in the prevention of in-stent restenosis. In the Boston Scientific sponsored TAXUS IV trial, which compared the TAXUS SR drug eluting stent on the Express-1 platform to an identical bare metal Express-1 stent, it was demonstrated that in-stent restenosis at 9 months can be reduced from 24.4% for the bare metal stent to 5.5% by using an equivalent drug eluting stent (Journal of Interventional Cardiology 2004; Vol. 17, No. 5, p279). Local drug delivery rather than systemic therapy has provided excellent results in the case of coronary drug-eluting stents; future therapies such as gene therapy and stem cell therapy require some form of local delivery device, as these therapies involve the time consuming production of expensive, minuscule quantities of molecules/compounds. A systemic non-efficient approach would not be cost effective for gene therapy, as most of the molecules/compounds would not reach the required target site - a different more efficient approach is required.

The state of the art at present for atherosclerosis, and in particular treating blocked coronary arteries, involves the implantation of a drug eluting coronary stent. This action re-establishes blood flow to ischemic areas of the heart muscle. However, there are certain situations caused by different stages of the disease or vascular disease affecting different blood vessels where a stent cannot be implanted. In these situations a different strategy must be adopted. Future therapies, such as biotherapeutic local delivery for molecular cardiology and molecular vascular intervention, are on the forefront of clinical medicine and promise to provide therapeutic treatment for the next generation of patients. These new treatment methods could make a difference to the quality of life of patients who have the following conditions:
- Chronic Total Occlusions (CTO).
   A CTO is a complete obstruction of an arterial lumen and it is estimated that 10-20% of all coronary angioplasty procedures involve a CTO (Freed and Safian, The Manual of Interventional Cardiology, 3rd ed; p287). CTOs can occur in other arteries, for example femoral arteries. A CTO in a femoral artery restricts blood flow to the remainder of the patient's leg and may cause critical limb ischemia, and consequently ulcerations and gangrene can occur and in some cases amputation is necessary. In addition slight angiogenesis (formation of new blood vessels) may occur allowing small amounts of blood to reach the lower leg. Angiogenesis in some cases may be crucial for survival. The process of angiogenesis can be artificially accelerated by injection of Vascular Endothelial Growth Factor (VEGF), this was demonstrated in an animal model of CTOs. Nikol et al. (Acta Physiologica Scandinavica 2002, Vol. 176, Iss. 2, p151) showed that injection of VEGF significantly increased the number of artery branches and the area of branches in a pig model of CTOs. With encouraging results from animal models it is expected that this form of gene therapy for CTOs will be transferred to a clinical application in the near future, if this occurs physicians would require a safe efficient catheter for delivery of the therapeutic solution.
- Peripheral Artery Disease (PAD)
   PAD is a condition similar to coronary artery disease. In PAD, fatty deposits build up in the inner linings of the artery walls, mainly in arteries leading to the kidneys, stomach, arms, legs and feet. This causes dysfunction of individual organs or limbs. PAD is slightly different to coronary artery disease as it affects arteries near to the surface of the body compared to the well-protected (from external mechanical loads) arteries of the heart. Stainless steel or cobalt chrome stents cannot be used safely in PAD because if they experience an excessive external load they will not retain their shape due to plasticity of the material. An external load in this case would cause an instantaneous obstruction within the artery lumen and consequent loss of blood flow. The challenging anatomy of peripheral arteries, the prevalence of long total occlusions, and a number of unique mechanical loads all lead to high restenosis rates in femoropopliteal and infrapopliteal interventions and patients with superficial femoral artery stenoses have patency rates of less than 50% at 1 to 3 years clinical follow-up (Radiology, 1994; 191; p727-733). Stents appear to be an inadequate treatment option for peripheral arteries and additional methods and treatment strategies for peripheral interventions that do not rely on a mechanical solution for the biological problem must be employed, i.e. local delivery of therapeutic products to these lesions.
- Stent edge restenosis and in-stent restenosis
   There is a potential for local biotherapeutic delivery to the edge of Bare Metal Stents (BMS) and Drug Eluting Stents (DES). In a study by Serruys *et al*. significant restenosis rates at the proximal edge of DES and BMS were reported in an IVUS study, at 6 months follow-up after stenting a significant decrease in proximal lumen area was observed for slow release, medium release TAXUS eluting stents and bare metal stents (Circulation 2004, Vol. 109, p627-633).
- Vulnerable plaque
   Vulnerable plaque is a type of lesion that is buried inside the artery wall and may not always bulge out and block blood flow; it is now an accepted fact that this type of plaque accounts for the vast majority of acute coronary syndromes (Cardiovascular Research 1999, Vol. 41, p323-333). Vulnerable plaque is asymptomatic and difficult to diagnose with present technology. However, advances in screening techniques and diagnostic technology (Virtual Histology IVUS and thermography catheters) allow these lesions to be identified. This type of lesion is non-stenotic and does not require a mechanical solution, it would be more advantages to change the function of the tissue by delivering a biotherapeutic solution to the lesion site.

Numerous catheter based local therapeutic delivery devices for the delivery of gene therapy products (or drugs) directly to target sites within a vessel or artery have been developed.

United States Patent No. 6,048,332 (Duffy, et al.) entitled "Dimpled porous infusion balloon" discloses drug delivery catheters that have dimpled porous balloons mounted onto the distal end of the catheter. In one embodiment, the balloons are adapted for delivering therapeutic agents to the tissue wall of a body lumen, and to this end include a plurality of dimples formed in the exterior surface of the balloon, with each dimple having at least one aperture through which a fluid delivered into the interior of the balloon can extravasate. It is understood that the balloons described therein provide, inter alia, increased coverage of the tissue wall to which the agent is being delivered and less traumatic contact between the agent being delivered and the tissue wall.

United States Patent No. 5,336,178 (Kaplan, et al.) discloses an intravascular catheter with an infusion array. An intravascular catheter provides means for infusing an agent into a treatment site in a body lumen and means for deploying the infusing means adjacent the treatment site, which operate independently of one another. In one embodiment, a flexible catheter body has an expansion member attached to its distal end in communication with an inflation passage, and an infusion array disposed about the expansion member in communication with one or more delivery passages. The infusion array includes a plurality of delivery conduits having laterally oriented orifices. The delivery conduits may be extended radially from the catheter body to contact a treatment site by expanding the expansion member with an inflation fluid. An agent may be introduced into the delivery passages and infused into the treatment site through orifices in the delivery conduits. The expansion member may be expanded for dilatation of the lumen before, during, or after infusion.

United States Patent No 6,369,039 (Palasis et al.) entitled "High efficiency local drug delivery" discloses a method of site-specifically delivering a therapeutic agent to a target location within a body cavity, vasculature or tissue. The method comprises the steps of providing a medical device having a substantially saturated solution of therapeutic agent associated therewith; introducing the medical device into the body cavity, vasculature or tissue; releasing a volume of the solution of therapeutic agent from the medical device at the target location at a pressure of from about 0 to about 5 atmospheres for a time of up to about 5 minutes; and withdrawing the medical device from the body cavity, vasculature or tissue. One problem with this device is its low delivery pressures.

The above are all examples of infusion catheters, with no needles involved. In vivo studies show that these catheters have inferior clinical results in comparison to other drug delivery methods. Infusion has been shown to be an inferior drug delivery method to needles.

United States Patent No. 5,112,305 (Barath, et al.) entitled "Catheter device for intramural delivery of therapeutic agents" discloses a method of treatment of an atherosclerotic blood vessel. Specifically, therapeutic agents are delivered by means of a specialized catheter system to the deeper layers of the vessel wall with only minimal interruption of the vessel endothelium. This system will allow high local concentrations of otherwise toxic agents directly at the site of an atherosclerotic plaque. The catheter system and method will deliver chemical agents intramurally at the precise vessel segment that is diseased but without allowing the agents to diffuse distally into the bloodstream. One embodiment disclosed employs a double lumen catheter that has additional tubular extensions projecting at various angles from the outer surface of the outermost lumen. By abruptly increasing the pressure in the outer lumen, the tubular extensions deliver the therapeutic agent to locations deep within the vessel wall.

This an example of an early device employing needles at a time when technology to join balloons and needles was undeveloped. Furthermore, the balloon needs to be inflated when it is not airtight due to the holes associated with the protrusions, which is not sensible and could cause problems with excessive therapeutic agents transferred to the blood stream rather than the target site. There may also be problems with balloon deflation.

United States Patent No. 5,873,852 (Vigil, et al.) entitled "Device for injecting fluid into a wall of a blood vessel", discloses a method and device for injecting fluid into a treatment area of a vessel wall. A first version of the device includes an inflatable balloon mounted on a catheter and a plurality of injectors extending outwardly and moving with the balloon. At least one fluid passageway connects each injector in fluid communication with a fluid source. During use of the device, the balloon is first positioned in a vessel proximate the treatment area. Next, the balloon is inflated to embed the injectors into the vessel wall. Subsequently, the fluid from the fluid source is introduced into the fluid passageway and through the injectors into the treatment area.

It will be appreciated therefore that the needles are free to cause damage to the endothelial surface upon delivery and retraction of the device.

United States Patent No. 5,354,279 (Hofling) entitled "Plural needle injection catheter" discloses a catheter for the injection of a fluid, for example, medicine, into body cavities such as veins or other hollow organs. The catheter is provided with a head which is insertable into the body cavity and includes hollow needles movably disposed therein between retracted and extended positions and with an operating mechanism mounted to the end of the catheter opposite the head and operatively connected to the needles for moving their front ends outwardly in contact with the walls of the body cavity for supplying the fluid or medicine through the hollow needles directly to the wall portions of the body cavities to be treated. A balloon may be disposed in front of the catheter head and may be inflated or deflated by way of a passage extending through the catheter. This needle injection catheter is awkward to use and requires additional steps that need precision control by the operator and may be prone to some form of error. Unpredictable advancement of the needle due to the difficult to control needle advancement mechanism might occur, and vessel perforations are possible, both of which are highly undesirable.

United States Patent No. 6,197,013 Reed, et al.) entitled "Method and apparatus for drug and gene delivery" discloses an apparatus and method for treating a patient. The apparatus includes a deployment mechanism having a surface. The apparatus also includes at least one probe disposed on the deployment mechanism surface. The probe extends between 25 microns and 1000 microns from the surface of the deployment mechanism. The apparatus also includes material coated on the probe. The method of treatment includes the steps of placing a material with a probe which extends less than 1000 microns from a surface of a deployment mechanism. Next, there is the step of inserting the probe into preferably a blood vessel of a patient. Then, there is the step of penetrating the interior wall of the vessel from the interior of the vessel with the probe by activating the deployment mechanism so the material can contact the vessel.

A problem with this arrangement is that the sharp probes on the outside of the stent or the catheter may cause damage during delivery or removal of the stent, although there is a mention of a protective sheath that is removed prior to dilation.

United States Patent No. 6,283,947 (Mirzaee) entitled "Local drug delivery injection catheter" discloses a catheter for injecting medication to a specific point within a patient comprises a drug delivery lumen extending from a proximal end of the catheter to an injection port. The catheter comprises a mechanism for angularly pushing the injection port outwardly away from the body of the catheter into an artery wall so that medication can be injected directly into the artery wall. The catheter comprises an injection port at or near the distal end thereof and a mechanism for directing the injection port angularly away from the central axis of the catheter and into the artery wall. (An injection port is a structure used for introducing medication or other material into a patient. The injection port typically is a hollow needle.) In one embodiment, the catheter includes a guide wire lumen for receiving a guide wire that enables a physician to direct the catheter to a desired location within the patient's vascular system. Also, in one embodiment, the catheter includes a plurality of needles, each of which may be manipulated at an angle outwardly from the central longitudinal axis of the catheter so that the needles can inject a drug or medication into the surrounding tissue. Prior to deployment of the needles, the needles are retained such that they lie substantially parallel to the longitudinal axis of the catheter. In one embodiment, a balloon is provided towards the distal end of the catheter for pushing the needles outwardly into the artery wall. In another embodiment, other mechanical means are provided for pushing the needles outwardly.

Problems experienced by this device include operational difficulties, difficulties with advancing sheath after use, and lack of flexibility.

United States Patent No. 6,494,862 (Ray, et al.) entitled "Substance delivery apparatus and a method of delivering a therapeutic substance to an anatomical passageway" discloses a catheter assembly having a balloon disposed at the distal end thereof. The balloon is capable of being inflated to selectively dilate from a collapsed configuration to an expanded configuration. A syringe assembly is in fluid communication with a delivery lumen of the catheter assembly for allowing a therapeutic substance to be injected into a tissue of a passageway. The syringe assembly includes a portion capable of pivoting from a first position towards a second position when the balloon is being inflated from the collapsed configuration to the expanded configuration. The portion of the syringe assembly is also capable of pivoting from the second position back towards the first position when the balloon is being deflated. One problem with this device is that the pivoting may cause ripping/damage of the inner artery wall.

United States Patent 6,695,830 (Vigil , et al.) entitled "Method for delivering medication into an arterial wall for prevention of restenosis" discloses a method for preventing a restenosis within a vessel wall, wherein a medicament is required to be delivered at predetermined locations into the vessel wall and allowed to subsequently disperse in a predetermined pattern. To deliver the medicament, a catheter with an expanding member is advanced into the vasculature of a patient until the expanding member is located as desired. The expanding member is then expanded to force dispensers into the vessel wall to the proper depth. A medicament is then pumped through the dispensers to create a plurality of equally spaced, localized medicinal deliveries which subsequently disperse to medicate an annulus shaped volume within the vessel wall.

It will be appreciated that current devices for delivering therapeutic agents to the arterial wall experience problems either with safety or efficiency. Furthermore, the current devices are limited in their areas of application. A further problem commonly experienced by current devices is incomplete balloon deflation or deflation failure. This causes a serious safety issue as it is essential that the balloon can deflate quickly and completely to allow removal of the catheter from the vessel without causing subsequent damage to the vessel wall.

Accordingly, what is required is a local catheter based therapeutic delivery device that allows treatment implements such as needles to be concealed when the catheter is being manoeuvred into position, to permit safe delivery of the device to the desired treatment area, without causing damage to the inner lining of the artery wall during delivery.

Naimark *et al* in US Patent Publication No. US 2004/0044308 describe an apparatus for the delivery of biologically active materials which includes a catheter, a balloon, microneedles on the balloon and which can further include a sheath. The sheath is described as being made of metals. One alternative!discussed is to make the sheath of expandable material. The sheath optionally has a plurality of ports for the microneedles or is made of a material capable of being punctured by those needles, The balloon of the Naimark *et al* device is inflated it moves out to contact the sheath and the sheath may, once contact is established, expand with the balloon. This construction can be seen for example from Figure 5a of that document. Having the sheath spaced radial y outward and apart from the microneedles (in Barath (above) outward of the blades; ensures protection for the vessel wall from scraping when the balloon is unexpanded.

Shaw *et al.* in International Patent Publication No. WO 2004/096097 describe an implantable and expandable medical device, which include a stent, a catheter having an inflatable balloon, and a sheath or sleeve, which are equipped with a cutting blade. One embodiment discussed is where a sheath or sleeve has at least one cutting blade mounted to the outer surface of the sheath (Fig. 26). The blades can be deployed anc/or retracted by a variety of mechanisms when the stent is expanded including folding and unfolding of the blades (Figs. 21 and 22), containing the blade within a trough on valley of the stent (Fig. 25), or where the blade is biodegradable thereby avoiding the need to retract the blade to prevent damage to the vessel wall. Other mechanisms used to retain the blades in an unexpanded state include the use of one or more retractable sheaths, sleeves or socks placed over the bladed sheath.

### Object of the Invention

It is an object of the invention to provide an efficient and effective catheter based local therapeutic device which may be adapted for the delivery of gene therapy products (or drugs) directly to target sites, and/or which may be provided with cutting implements which can be used to treat a site within the body.

It is a further object of the invention to provide a local catheter based therapeutic delivery device capable of use in a number of product applications.

It is a further object of the invention to provide a delivery device which can be used at more than one site of treatment within a vessel/artery. This feature is particularly useful in diffuse peripheral disease or for arteries with numerous vulnerable plaques.

It is a further object of the device to provide a delivery device which experiences quick and safe deflation after use.

It is a further object of the invention to provide a delivery device with sufficient flexibility so as to allow the catheter to navigate tortuous arteries.

It is a further object of the invention to provide a delivery device wherein drugs may be delivered (and thus distributed) evenly compared to catheters available at present.

It is a further object of the invention to provide an improved cutting balloon for use in opening blocked vessels.

### Summary of the Invention

Accordingly, there is provided a device for treating a target area of a vessel wall of a vessel within a human or animal body according to independent claim 1.

Preferably the expandable portion is a balloon.

In one embodiment the treatment implements may be blades for cutting or scoring the vessel wall. Alternatively, the treatment implements may take a different form or example needles (such as hollow needles or micro-needles) wherein the device may act as a drug delivery device for the delivery of therapeutic substances to the vessel wall. When needles are used, preferably the device further comprises a drug delivery system in fluid communication with the needles for delivery of therapeutic compound through the needles into the vessel wall. The drug delivery system may comprise a plurality of reservoirs in the protective sheath. Alternatively, the drug delivery system may comprise a (multi-lumen) supply hose connected via (flexible) tubing to the needles.

Preferably the protective sheath comprises an elastic polymer, such as silicone. Preferably the protective sheath has defined therein a plurality of holes in which the treatment implements are seated.

The device may further comprise at least one marker (such as a radiopaque marker) to aid positioning of the device.

According to the invention there is further provided a sheath for fitting to a balloon catheter for treating a target area of a vessel wall of a vessel within a human or animal body, as defined by independent claim 12.

It will be appreciated in such embodiments that the sheath acts as a carrier for the treatment implements, which may be coupled or mounted on or within the sheath. Preferably the sheath comprises an elastic polymer, such as silicone.

In one embodiment, the treatment implements may be one or more needles for example hollow needles. The sheath may then further comprise:
an inner sheath comprising an outer surface on which a plurality of reservoirs are provided for storing therapeutic compound; and
an outer sheath positioned over the inner sheath;
wherein the needles each comprise a base portion and an injector portion, and wherein each base portion is located over a reservoir on the outer surface of the inner sheath, and wherein each injector portion extends radially outwards from the inner sheath and is received through cooperating holes defined within the outer sheath.
When treatment implements are needles, the sheath may be used to convert a standard balloon catheter into a catheter based drug deliver device.

In an alternative embodiment the treatment implements may be cutting implements for example blades, or microsurgical scalpels. The sheath preferably contains a number of microsurgical scalpels on its outer surface. These scalpels may be initially concealed from the artery wall by the external contours of the sheath.

The sheath may comprise at least one protruberance on its outer surface, wherein in each protruberance extends further radially outwardly from the outer surface of the sheath than each cutting implement.

Preferably each protruberance is collapsible. In a preferred embodiment each protruberance has a hollow internal pocket (a hollow centre), wherein in the balloon's expanded configuration the deformation of the sheath causes the pocket to flatten out thereby reducing the size of the protruberance in the radial direction to expose each cutting implement. The protruberance therefore becomes flattened as the sheath deforms with inflation of the balloon. When the balloon is inflated the contours of the sheath become smooth and the cutting edges are exposed. Moreover the sheath allows optimum balloon folding and minimum balloon withdrawal resistance leading to a safer and easier to use device. The silicone sheath has a number of functions, (i) it protects the artery wall from the scalpel blades when the catheter is being manoeuvred in to position, (ii) it prevents balloon/blade direct contact so the balloon cannot be dissected by a blade, (iii) keeps all the blades perpendicular to the balloon at all times, (iv) aids deflation of the balloon to its original profile which subsequently reduces balloon withdrawal resistance, (v) the sheath allows optimum folding of the balloon which will reduce the profile of the catheter when compared to present technology.

It will be appreciated that when the treatment implements are blades, the sheath may be used to convert a standard angioplasty balloon into a cutting balloon.

The sheath may further be provided with at least one marker such as a radiopaque marker to aid positioning of the sheath.

Accordingly, there is provided a local catheter based treatment device for use as a therapeutic substance delivery device or a cutting device, based on a technology platform that utilises an efficient and safe technology to treat sites of disease/damage within a blood vessel wall. The technology is a catheter-based system that utilises the material properties of a soft sheath (made from, for example, silicone /or custom microstructural material) to conceal treatment implements (such as injection needles) from the artery wall when the catheter is being advanced to its site of use.

When the catheter is located at its intended site of use a balloon is inflated. In an embodiment wherein the treatment implements are needles, this forces a series of needles outwards in the radial direction; the balloon expansion causes the sheath to stretch over the balloon, and the needles, which are located between the balloon and sheath, are pushed through holes located in the sheath and onwards into the site of disease or desired area of drug delivery in the artery wall.

The device relies on this principle to conceal the needles initially and secondly to utilise the incompressible material properties of the sheath to allow the needles to be exposed at the site of therapeutic delivery when the balloon is inflated. The technology offers a safe methodology to deliver therapeutic agents as the catheter will cause minimal damage to the artery wall when it is being placed in position.

A diffuse needle arrangement allows the drugs to be distributed evenly compared to catheters available at present. Minimum damage is caused to the artery wall by this method thus neointimal hyperplasia should not be a significant problem with the device of the present invention.

It will be appreciated that the device can be used at more than one site as the sheath causes the balloon and the needles to retract into their original position. Following this, the device could be moved to the next site of treatment. This feature could be useful in diffuse peripheral disease or for arteries with numerous vulnerable plaques. This feature also reduces the balloon withdrawal resistance of the device.

It will further be appreciated that the sheath also protects the balloon against contact with the implements. Contact between the implements and the balloon is undesirable as could cause puncturing of the balloon.

The primary advantage of the device of the present invention is the manner in which the treatment implements are concealed within the catheter and the manner in which the material properties of the sheath are used to reveal the implements at the correct location.
Moreover, using this device, the method of drug delivery is more efficient than methods available at present.

It is these two aspects that differentiate the invention from products available, and patented products that are not in clinical use at present. Previous designs incorporated exposed needles, which could cause damage to the artery wall, and previous local drug delivery catheters were never very efficient, delivering only approximately 15% of the drug to the desired area.

The approach taken by the device of the invention will always cause balloon deflation after a procedure, as the elastic sheath will produce automatic balloon deflation and retraction of the needles. This removes any doubt of issues of balloon deflation. Prior art devices do not have this fail-safe mechanism.

Further differences between the invention and the prior art include:
1. The sleeve always fits tightly on the balloon in both the retracted and expanded positions.
2. The elastic material is used to conceal implements
3. The sheath can be retrofitted to any balloon catheter.

Furthermore, the invention may be used as a platform technology for a number of different applications, either as a stand alone device or as an additional feature of a current procedure e.g. a module to prevent proximal or distal restenosis during delivery of a drug eluting stent.

The technology could provide a significant commercial return as current devices for delivering therapeutic agents to the arterial wall, and devices for dilation of diseased vessels are not as safe or as efficient as the proposed platform technology, furthermore the current devices are limited in their areas of application while this present technology platform has been designed so that a number of product applications are possible.

It will be appreciated that the geometry and design of the device may be adapted to suit its intended application. For example, when used as a chronic total occlusion catheter, all the needles will be weighted towards the front of the catheter, the profile will be modified slightly and a specific balloon geometry will be used to account for the lesion geometry.

The device of the invention may also be used for local biotherapeutic delivery to the edge of Bare Metal Stents (BMS) and Drug Eluting Stents (DES).

A device according to the present invention may be incorporated a stent delivery catheter. The design of this module will not compromise the cross-ability or the profile of the stent delivery catheter. On BMSs, use of the invention in this manner may reduce in-stent restenosis. This module would allow direct injection into the artery wall of anti proliferative drugs without the need to develop complex and costly drug eluting polymer coatings.

For a drug delivery module located on a DES it is expected that the material properties or geometry will have to be altered slightly to match that of the stent expansion so that a single balloon could be used for the entire delivery (stent and drugs), the drugs could be injected as the stent is being held in place by the cardiologist.

The present invention could be used to deliver the biotherapeutic solution to the lesion site.

There is further disclosed an exemplary method of treating one or more target areas of a vessel wall within a human or animal body, the method comprising the steps of:
a) providing a device comprising:
   an expandable portion for radially expanding the device from a contracted configuration allowing travel within the vessel to the target area to an expanded configuration allowing treatment of the target area;
   a protective sheath stretch-fitted over the expandable portion to exert a compressive force on the expandable portion for radially contracting the device from its expanded configuration to its contracted configuration, and for exerting a compressive force on the expandable portion in its contracted configuration; and
   at least two spaced apart treatment implements extending radially outwardly from the expandable portion, wherein in the device's contracted configuration the implements are shielded within the protective sheath, and in its expanded configuration the thickness of the sheath decreases to expose the implements for contact with the target area of the vessel wall;
b) inserting the device in its contracted configuration into the interior of the vessel;
c) advancing the device through the vessel to reach the target area;
d) providing an expansive force to expand the expandable portion to expose the implements for contact with the vessel wall;
e) removing the expansive force to allow the compressive force of the sheath to radially contract the device from its expanded configuration to its contracted configuration;
f) repeating steps c) to e) until all target areas have been treated; and
g) withdrawing the device from the vessel.

The method further comprises, after exposing the implements for contact with the vessel wall, the step of delivering therapeutic compound through the treatment implements into the vessel wall.

When used as a vulnerable plaque catheter, modifications will need to be made to allow the needles to enter the plaque cap with minimal damage caused to the fibrous cap of the lesion.

### Brief Description of the Drawings

The present invention will now be described in greater detail with reference to the accompanying drawings in which:
Figure 1 is a representation of a device according to the present invention.
Figure 2 is a sectional representation of a device according to one embodiment of the invention during operation in-vivo within a vascular cavity.
Figure 3a is a side cross sectional view of the device of Figure 2 pre balloon deployment.

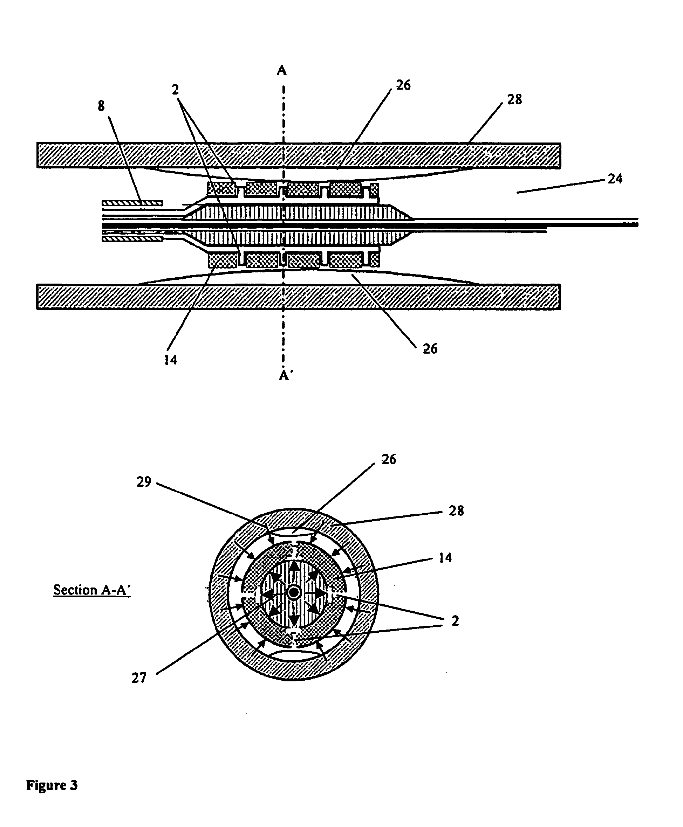

Figure 3a is an end cross-sectional view of the device of Figure 3a taken along line A-A' in Figure 3a.

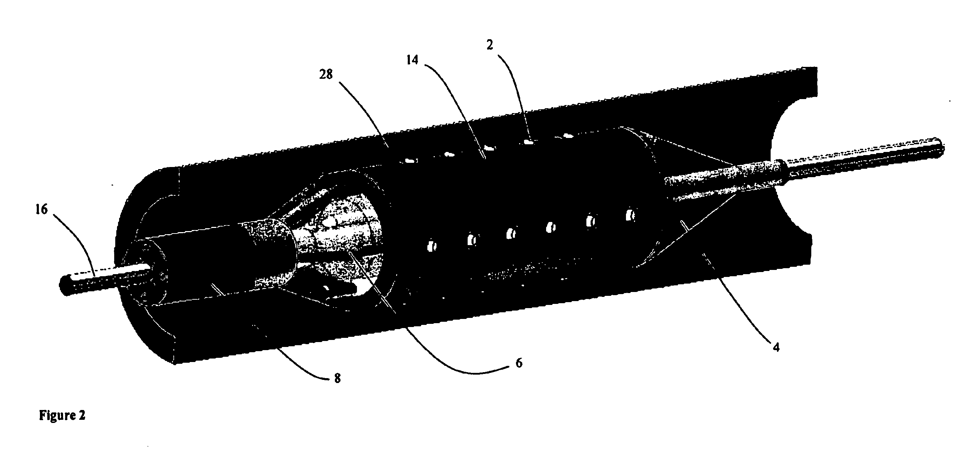

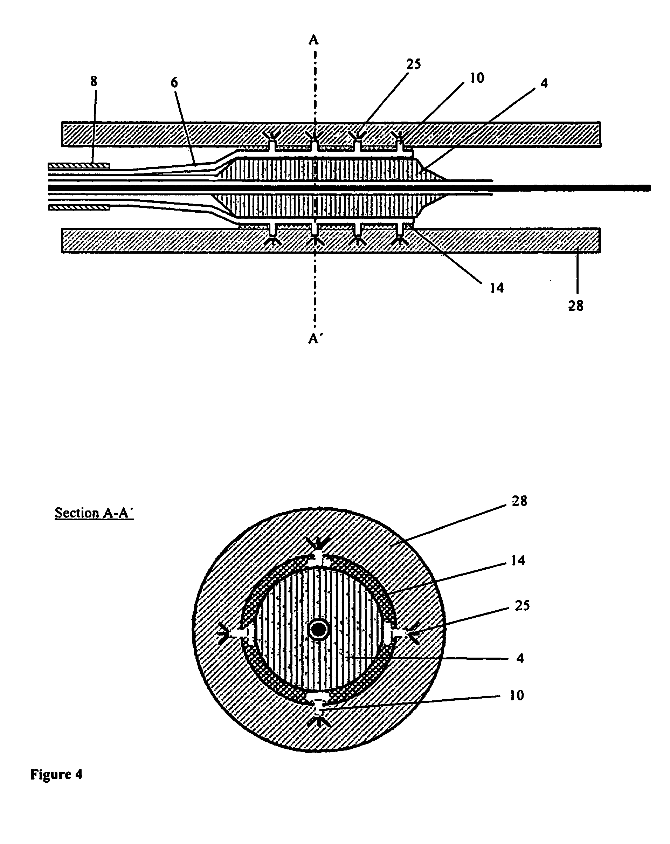

Figure 4a is a side cross sectional view of the device of Figure 2 post balloon deployment and drug delivery.
Figure 4a is an end cross-sectional view of the device of Figure 4a taken along line A-A' in Figure 4a.

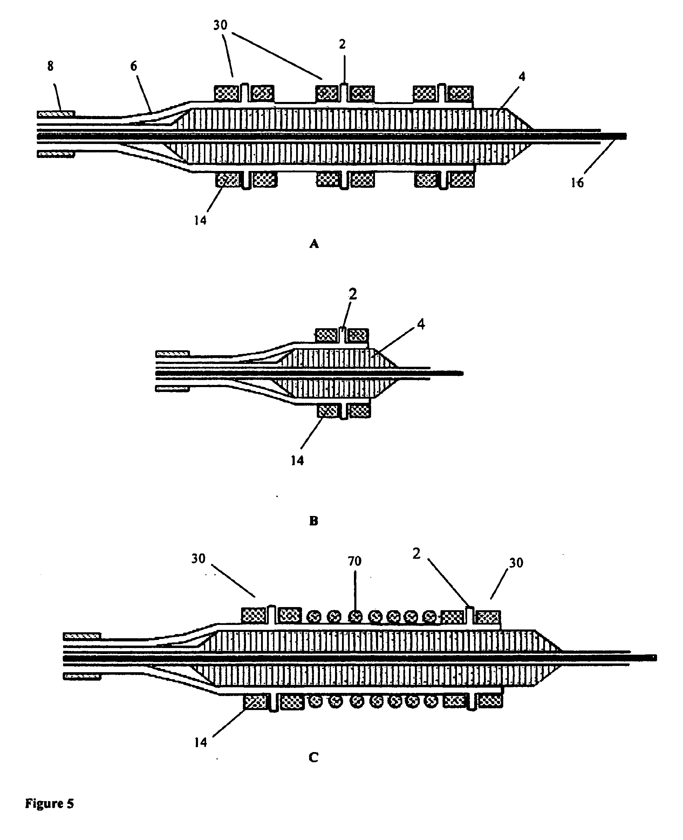

Figure 5 is a set of perspective views of three further embodiments of devices according to the invention.

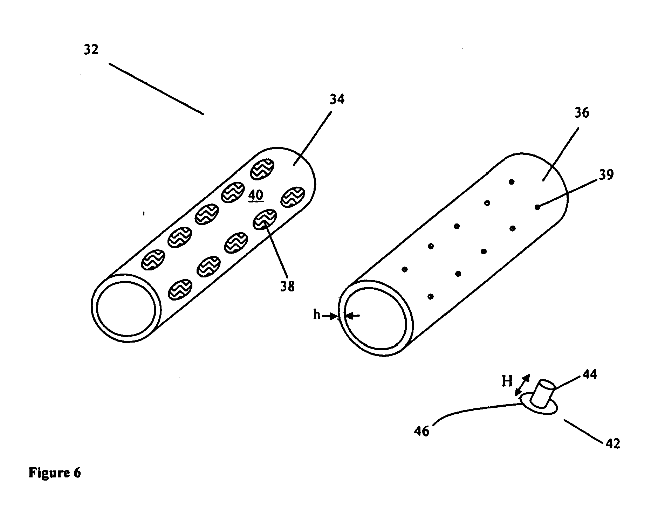

Figure 6 is a perspective representation of a sheath according to one embodiment of the invention.

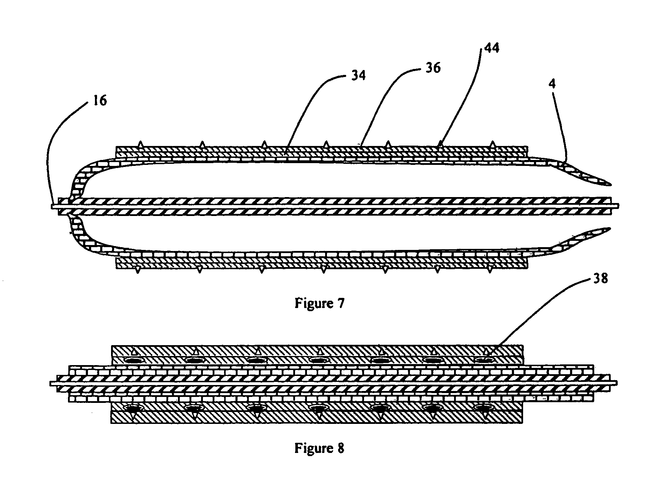

Figure 7 is a cross-sectional view of a device in accordance with the invention in its expanded configuration.
Figure 8 is a cross-sectional view of the device of Figure 7 in its retracted configuration.

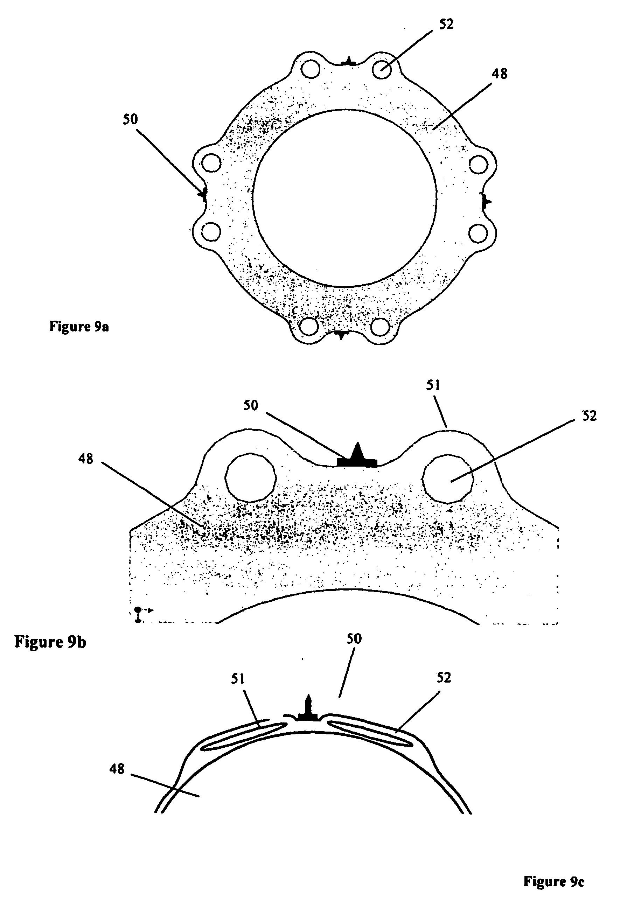

Figure 9a is a cross-sectional view of a cutting sheath according to one embodiment of the invention.
Figure 9b is a close-up view of a blade region of the sheath of Figure 9a.
Figure 9c is cross-sectional view of a portion of the sheath of Figure 9a in its deformed state with the blade exposed.

### Detailed Description of the Drawings

Presented in the drawings is a catheter based device for the treatment of internal body cavities such as arteries/veins or other hollow organs in accordance with the present invention. Also presented is a retrofit sheath and a sheath loading device in accordance with the present invention.

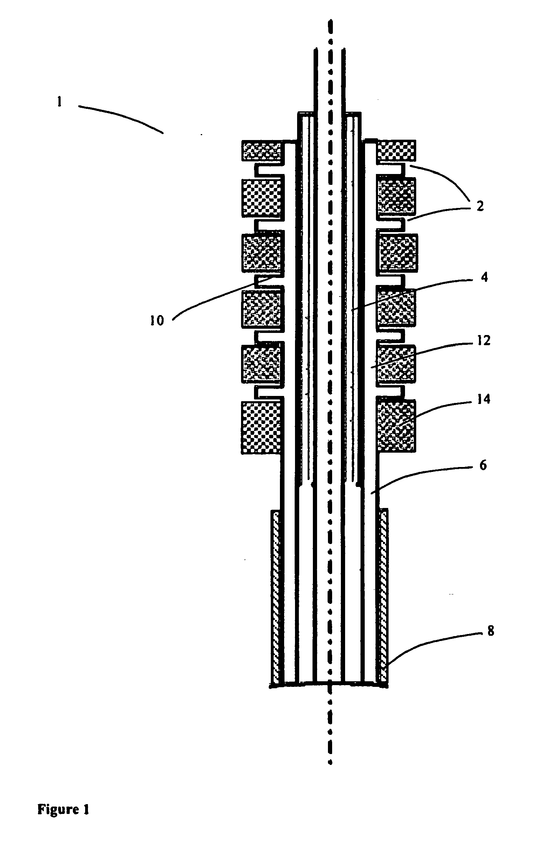

Figure 1 shows a catheter based drug delivery device 1 in accordance with one embodiment of the invention. The device is insertable into a vasculature via a guide wire (as shown in Figure 2), includes micro-needles 2 that have two positions, a retracted position and an extended position. These needles 2 are mounted on the surface of a balloon catheter 4 and connected via flexible tubing 6 to a multi-lumen supply hose 8. The needles/micro-needles or stems (for directly injecting medicine(s)) attached to hollow needle base reservoirs 12. The needle stems 10 project outward from the reservoir 12 and are protected within a rubber sleeve or sheath 14. Upon inflation of the balloon 4 the needles 2 move outwards (in the radial direction), and stretching and compressing of the protective sheath 14 occurs, which in turn acts to expose the needles 2. The needles 2 when exposed can become embedded in the wall of the body cavity. Drugs may be delivered locally, for example to the diseased vessel wall, when the balloon 4 is inflated and subsequently when the needles 2 are embedded in the body cavity such as an artery wall. When balloon deflation occurs the needles 2 retract under the canopy of the sheath 14. The deflated assembly can now be safely removed from the body via a guide wire 16. During this procedure, the needles 2 are concealed and will not cause damage to the endothelium upon insertion and removal of the device.

At rest, the inner diameter of the elasticised sheath 14 is dimensioned so as to be smaller than the outer diameter of a balloon catheter 4 in its collapsed state. This ensures a tight fit between the sheath and balloon at all times when the sheath is loaded on the balloon. The sheath must therefore be stretch-fitted onto the balloon catheter. The elastic nature of the sheath ensures that the sheath will exert a compressive force on the balloon at all times. The balloon is thus maintained in its deflated state at all times except when a greater opposite force is exerted on the sheath by the balloon under the influence of air/fluid introduced under pressure into the balloon to inflate it.

In all embodiments the expandable member (balloon) will generally have a collapsed configuration where there is substantially no air or other inflating fluid in the balloon. Generally the balloon will also be in a folded configuration when collapsed. Desirably the compressive force of the sheath acts on the balloon in its folded configuration. The sheath acts to bias the balloon toward its folded configuration.

When the balloon is inflated, it is desirable that the sheath causes a tight seal between the needles and the artery wall allowing leak-free delivery. This seal may be achieved by selecting a soft material for the sheath such as a silicone material. Other suitable materials for the sheath include polyurethane.

As mentioned, elastic properties of the sheath cause the needles to retract once the balloon is deflated. This allows the device to return to its original configuration and allows the device to be used at multiple sites during the one procedure.

The protective foam-rubber cover or sheath 14 is shown in Figure 2. The selected material is both flexible and compressible enough to allow the needle stems 2 to expose upon balloon deployment, but more importantly provides and aids timely retraction and protection of the needle stems when balloon deflation occurs. This is particularly important for safe insertion and timely removal of the device.

Figures 3 and 4 depict sectional schematics of the device during operation and in-vivo, within a partially occluded vascular cavity 24. In Figure 3, a plaque 26 is shown to have occurred locally around the inner cavity wall 28 causing partial occlusion. The device is shown placed in situ. Arrows 27 represent the balloon deployment force while arrows 29 represent the reaction force of the compressing sleeve.

Figures 3 and 4 illustrate one of the key features of the device which is shown in operation during mid and post deployment. As shown, the balloon pressure 27 causes the micro-needles 2 to move outward in the radial direction. Due to the compressive force and the circumferential stretch the protective sheath 14 is compressed (generally compression of the sheath will be due to the Poisson effect) thus exposing the micro-needle stems 10 allowing drug delivery (indicated by lines 25) directly into the plaque 26 on the cavity wall 28.

Figure 5 depicts three embodiments of devices in accordance with the invention, labeled A-C. Embodiment A is a particularly flexible embodiment based on a modular design where the sheath 14 is provided with a plurality of rings 30 of material. These rings 30 may be completely separate from one another or may be connected by one or more interconnecting links. Embodiment B has a short balloon 4, and the sheath 14 comprises treatment implements 2 adjacent the balloon's leading end. This embodiment is most suitable to treating chronic total occlusions, as therapeutic delivery will occur as close as is possible to the blockage. This ensures that the therapeutic solution could instigate remodeling of the vasculature as close as possible to the diseased section, for example angiogenesis promoters would allow collaterals to form immediately upstream of the blockage ensuring that all areas of the limb/organ are supplied with blood flow. Embodiment C is a proximal and distal restenosis module suitable for attachment to a stent-loaded catheter. A stent 70 is shown in situ around the central portion of the balloon 4, between the sheath rings 30 which are confined to either end of the balloon 4. This module has the capability to deliver therapeutic agents to the artery wall immediately distal, proximal or both, of the area where a stent is being implanted, this would remove or reduce the risk of edge restenosis.

Figure 6 shows a retrofit sheath 32 according to one embodiment of the invention. The sheath is a two part sheath comprising an inner 34 and outer sheath 36. The inner sheath 34 has concave reservoirs 38 in (for example molded into) its outer surface 40, while the outer sheath 36 has small holes 39 defined within it to allow the needles sit within. A needle/plate assembly 42 sits beneath the outer sheath 36. The height h of the outer sheath 36 is greater than the height H of the needles 44. Once the needle assemblies 42 are in place, with the plates 46 positioned over the concave reservoirs, the outer sheath 36 is mounted over the inner sheath 34 to form the completed sheath shown in Figures 7 and 8. When the sheath is loaded on the catheter the therapeutic solution is stored within the sheath in the concave cavities/reservoirs. After the catheter has been maneuvered to the site of vascular disease the balloon is dilated. Upon dilation of the balloon, the sheath is stretched and the cavities within the sheath reduce in volume. This decrease in volume causes the therapeutic solution to be expelled from the reservoir and delivered to the site of disease.

Figure 7 shows the retrofit sheath of Figure 6 loaded onto a balloon catheter, the balloon catheter in its expanded configuration. Figure 8 shows the same arrangement with the catheter in its retracted configuration.

In the case of a retro fit sheath to be used as a cutting device, a simplified sheath may be employed. Figures 9a-9c show a retrofit cutting sheath 48 wherein the treatment implements are blades 50, which may be microsurgical scalpels. The scalpels are initially concealed from the artery wall by the external contours of the sheath 48, this allows the catheter to be navigated to the diseased portion of an artery without damaging the healthy vessel wall. It is the protruberances or bumps 51 in the sheath 48 as shown in Figure 9, which allow the blades 50 to be concealed from the artery wall, prior to and after use. When the sheath is positioned on a balloon and the balloon is inflated, the holes 52 in the bumps 51 flatten out as shown in Figure 9c, the contours of the sheath 48 become smooth and the cutting edges of the blades 50 are exposed. These blades 50 then contact the stenotic artery wall and allow an atherosclerotic lesion to be dilated in a controlled fashion. This approach allows the balloon expansion force to be concentrated at a number of discrete points and difficult lesions can be dilated successfully at lower pressures (4 - 8 atm or 4 - 8 x 10⁵ Pa).

The sheath or sleeve 50 can be adapted to be retrofitted to any balloon catheter. In the case of the present invention the sheath 48 is made of an elastic material and it will be appreciated that concealment of implements 50 is achieved because of the elastic properties of the sleeve 48. The holes 52 in the sheath will allow exposure of the blades 50 upon dilation of the balloon and deformation of the sheath; this is shown in the final schematic of Figure 9. There could be many other designs of cutting sheath, including a discontinuous tube that is joined at discrete points, similar to the needle version shown in Figure 5a.

Radiopaque markers could be added to the sheath to aid placement during the procedure (balloon angioplasty procedure).

The words "comprises/comprising" and the words "having/including" when used herein with reference to the present invention are used to specify the presence of stated features, integers, steps or components but does not preclude the presence or addition of one or more other features, integers, steps, components or groups thereof.

## Claims

1. A device (I) for treating a target area of a vessel wall of a vessel within a human or animal body, the device comprising:
a) an expandable portion (4) for radially expanding the device from a contracted configuration allowing travel within the vessel to the target area to an expanded configuration allowing treatment of the target area;
b) at least two spaced apart treatment implements (2, 50) extending radially outwardly from the expandable portion;
c) a protective sheath (14);
and **characterised in that**:
the protective sheath (14) is adapted to be stretch- fitted over the expandable portion to exert a compressive force on the expandable portion for radially contracting the device from its expanded configuration to its contracted configuration, and for exerting a compressive force on the expandable portion in its contracted configuration;
wherein in the device's contracted configuration the implements are shielded within the protective sheath, and in its expanded configuration the thickness of the sheath decreases to expose the implements for contact wit the target area of the vessel wall.

2. A device according to claim 1 wherein the expandable portion is a balloon.

3. A device according to claim 1 or claim 2 wherein the treatment implements are blades (50).

4. A device according to any preceding claim wherein the treatment implements are needles (2).

5. A device according to claim 4 further comprising a delivery system in fluid communication with the needles for delivery of therapeutic compound through the needles into a vessel wall,

6. A device according to claim 5 wherein the drug delivery system comprises a plurality of reservoirs (12) in the protective sheath.

7. A device according to claim 5 wherein the drug delivery system comprises a supply hose (8) connected via tubing (22) to the needles (2).

8. A device according to any preceding claim wherein the protective st eath comprise an elastic polymer.

9. A device according to claim 8 wherein the elastic polymer comprises silicone,

10. A device according to any preceding claim wherein the protective sheath has defined therein a plurality of holes in which the treatment implements are seated.

11. A device according to any preceding claim, further comprising at least one marker to aid positioning of the device.

12. A sheath (32, 48) for fitting to a balloon catheter for treating a target area of a vessel wall of a vessel within a human or animal body, the sheath adapted to be stretch-fitted over the balloon to exert a compressive force on the balloon for radially contracting the balloon from its expanded configuration to its contracted configuration the sheath comprising:
at least two spaced apart treatment implements (42,50), the sheath **characterised in that** the at least two spaced apart treatment implements (42, 50) are mounted within the sheath so as to extend radially outwardly from the balloon, wherein in the baloon's contracted configuration the implements are shielded within the sheath, and in its expanded configuration the thickness of the sheath decreases to expose the implements for contact with the target area of the vessel wall.

13. The sheath of claim 12 wherein the treatment implements are needles (42).

14. The sheath of claim 13 further comprising:
an inner sheath (34) comprising an outer surface (40) on which a plurality of reservoirs (38) are provided for storing therapeutic compound;
an outer sheath (36) positioned over the inner sheath (34);
wherein the needles (42) each comprise a base portion (46) and an injector portion (44), and wherein each base portion is located over a reservoir on the outer surface of the inner sheath, and wherein each injector portion extends radially outwards from the inner sheath and is received through cooperating holes (39) defined within the outer sheath.

15. The sheath of claim 12 wherein the treatment implements are cutting implements (50).

16. The sheath of claim 13 wherein the sheath comprises at least one protruberance (51) on its outer surface, wherein in the balloon's contracted configuration each protruberance extends further radially outwardly from the outer surface of the sheath than each cutting implement.

17. The sheath of claim 16 wherein each protruberance is collapsible.

18. The sheath of claim 16 or claim 17 wherein each protruberance has a hallow internal pocket, wherein in the balloon's expanded configuration the deformation of the sheath causes the pocket to flatten out thereby reducing the size of the protruberance in the radial direction to expose each cutting implement.

## Patentansprüche

1. Vorrichtung (1) zum Behandeln eines Zielgebietes einer Gefäßwand eines Gefäßes in einem menschlichen oder tierischen Körper, wobei die Vorrichtung Folgendes aufweist:
a) einen erweiterbaren bzw. expandierbaren Teil (4) zum radialen Erweitern der Vorrichtung von einer verengten bzw. kontrahierten Konfiguration, die die Fortbewegung innerhalb des Gefäßes zu dem Zielgebiet gestattet, zu einer expandierten Konfiguration, die die Behandlung des Zielgebietes zulässt;
b) mindestens zwei voneinander beabstandete Behandlungswerkzeuge bzw. Behandlungsinstrumente (2, 50), die sich radial nach außen von dem expandierbaren Teil erstrecken;
c) eine Schutzhülle (14);
und **dadurch gekennzeichnet, dass**
die Schutzhülle (14) geeignet ist, um über den expandierbaren Teil stretchgepasst zu werden, um eine komprimierende Kraft auf den expandierbaren Teil auszuüben, um die Vorrichtung radial von ihrer expandierten Konfiguration zu ihrer kontrahierten Konfiguration zu komprimieren, und um eine komprimierende Kraft auf den expandierbaren Teil in seiner kontrahierten Konfiguration auszuüben;
wobei die Instrumente in der kontrahierten Konfiguration der Vorrichtung innerhalb der Schutzhülle abgeschirmt sind, und wobei die Dicke der Hülle in ihrer expandierten Konfiguration abnimmt, um die Instrumente für den Kontakt mit dem Zielgebiet der Gefäßwand freizulegen.

2. Vorrichtung gemäß Anspruch 1, wobei der expandierbare Teil ein Ballon ist.

3. Vorrichtung gemäß Anspruch 1 oder Anspruch 2, wobei die Behandlungsinstrumente Schneiden (50) sind.

4. Vorrichtung gemäß einem vorhergehenden Anspruch, wobei die Behandlungsinstrumente Nadeln (2) sind

5. Vorrichtung gemäß Anspruch 4, welche ferner ein Liefersystem in Strömungsmittelverbindung mit den Nadeln aufweist zur Lieferung einer therapeutischen Zusammensetzung durch die Nadeln in eine Gefäßwand.

6. Vorrichtung gemäß Anspruch 5, wobei das Arzneimittelliefersystem eine Vielzahl von Reservoirs bzw. Behältern (12) in der Schutzhülle aufweist.

7. Vorrichtung gemäß Anspruch 5, wobei das Arzneimittelliefersystem einen Zulaufschlauch (8) aufweist, der über Schläuche (22) mit den Nadeln (2) verbunden ist.

8. Vorrichtung gemäß einem vorhergehenden Anspruch, wobei die Schutzhülle aus einem elastischen Polymer besteht.

9. Vorrichtung gemäß Anspruch 8, wobei das elastische Polymer aus Silikon besteht.

10. Vorrichtung gemäß einem vorhergehenden Anspruch, wobei in der Schutzhülle eine Vielzahl von Löchern definiert ist, in denen die Behandlungsvorrichtungen gelegen sind.

11. Vorrichtung gemäß einem vorhergehenden Anspruch, welche ferner zumindest eine Markierung aufweist, um beim Positionieren der Vorrichtung zu helfen.

12. Hülle (32, 48) zum Anpassen an einen Ballonkatheter zum Behandeln eines Zielgebietes einer Gefäßwand eines Gefäßes in einem menschlichen oder tierischen Körper, wobei die Hülle ausgelegt ist, um über den Ballon stretchgepasst zu werden, um eine Kompressionskraft auf den Ballon auszuüben, um den Ballon von seiner expandierten Konfiguration zu seiner kontrahierten Konfiguration radial einzuengen,
wobei die Hülle Folgendes aufweist:
mindestens zwei voneinander beabstandete Behandlungsinstrumente (42, 50), wobei die Hülle **dadurch** charakterisiert ist, dass die mindestens zwei voneinander beabstandeten Behandlungsinstrumente (42, 50) innerhalb der Hülle angebracht sind, um sich von dem Ballon radial nach außen zu erstrecken, wobei die Vorrichtungen in der kontrahierten Konfiguration des Ballons in der Hülle abgeschirmt sind, und wobei die Dicke der Hülle in ihrer expandierten Konfiguration abnimmt, um die Instrumente für den Kontakt mit dem Zielgebiet der Gefäßwand freizulegen.

13. Hülle gemäß Anspruch 12, wobei die Behandlungsinstrumente Nadeln (42) sind.

14. Hülle gemäß Anspruch 13, welche ferner Folgendes aufweist:
eine innere Hülle (34), welche eine Außenoberfläche (40) aufweist, auf welcher eine Vielzahl von Reservoirs bzw. Behältern (38) zum Speichern einer therapeutischen Zusammensetzung vorgesehen ist;
eine äußere Hülle (36), die über der Innenhülle (34) positioniert ist;
wobei die Nadeln (42) jeweils einen Basisteil (46) und einen Injektor- bzw. Einspritzteil (44) aufweisen, und wobei jeder Basisteil über einem Behälter auf der Außenoberfläche der Innenhülle gelegen ist, und wobei sich jeder Einspritzteil radial nach außen von der Innenhülle erstreckt und durch zusammenwirkende Löcher (39), die in der Außenhülle definiert sind, aufgenommen wird.

15. Hülle gemäß Anspruch 12, wobei die Behandlungsinstrumente Schneideinstrumente (50) sind.

16. Hülle gemäß Anspruch 13, wobei die Hülle auf ihrer Außenoberfläche mindestens einen Vorsprung (51) aufweist, wobei sich jeder Vorsprung in der kontrahierten Konfiguration des Ballons von der Außenoberfläche der Hülle weiter radial nach außen erstreckt als jedes Schneideinstrument.

17. Hülle gemäß Anspruch 16, wobei jeder Vorsprung zusammenklappbar ist.

18. Hülle gemäß Anspruch 16 oder Anspruch 17, wobei jeder Vorsprung eine hohle Innentasche besitzt, wobei die Verformung der Hülle in der expandierten Konfiguration des Ballons bewirkt, dass sich die Tasche abflacht, wodurch die Größe des Vorsprungs in der radialen Richtung verringert wird, um jedes Schneideinstrument freizulegen.

## Revendications

1. Dispositif (1) pour le traitement d'une zone cible d'une paroi vasculaire d'un vaisseau dans un corps humain ou animal, le dispositif comprenant :
a) une partie extensible (4) pour étendre radialement le dispositif à partir d'une configuration contractée, permettant le déplacement dans le vaisseau vers une zone cible, vers une configuration étendue permettant le traitement de la zone cible ;
b) au moins deux outils de traitement espacés entre eux (2, 50) s'étendant radialement vers l'extérieur à partir de la partie extensible ;
c) une gaine de protection (14) ;
et **caractérisé en ce que** :
la gaine de protection (14) est apte à être montée par étirement sur la partie extensible pour exercer une force de compression sur la partie extensible pour contracter radialement le dispositif à partir de sa configuration étendue vers sa configuration contractée, et pour exercer une force de compression sur la partie extensible dans sa configuration contractée ;
dans lequel dans la configuration contractée du dispositif les outils sont protégés dans la gaine de protection, et dans sa configuration étendue l'épaisseur de la gaine diminue pour exposer les outils pour un contact avec la zone cible de la paroi vasculaire.

2. Dispositif selon la revendication 1, dans lequel la partie extensible est un ballonnet.

3. Dispositif selon la revendication 1 ou 2, dans lequel les outils de traitement sont des lames (50).

4. Dispositif selon l'une quelconque des revendications précédentes, dans lequel les outils de traitement sont des aiguilles (2).

5. Dispositif selon la revendication 4, comprenant en outre un système d'alimentation en communication de fluide avec les aiguilles pour fournir un composé thérapeutique par l'intermédiaire des aiguilles dans une paroi vasculaire.

6. Dispositif selon la revendication 5, dans lequel le système d'alimentation en médicament comprend une pluralité de réservoirs (12) dans la gaine protectrice.

7. Dispositif selon la revendication 5, dans lequel le système d'alimentation en médicament comprend un tuyau d'alimentation (8) connecté par l'intermédiaire d'un tubage (22) aux aiguilles (2).

8. Dispositif selon l'une quelconque des revendications précédentes, dans lequel la gaine de protection comprend un polymère élastique.

9. Dispositif selon la revendication 8, dans lequel le polymère élastique comprend du silicone.

10. Dispositif selon l'une quelconque des revendications précédentes, dans lequel une pluralité de trous est définie dans la gaine de protection, dans lesquels sont placés les outils de traitement.

11. Dispositif selon l'une quelconque des revendications précédentes, comprenant en outre au moins un marqueur pour aider au positionnement du dispositif.

12. Gaine (32, 48) destinée à être montée sur un cathéter à ballonnet pour le traitement d'une zone cible d'une paroi vasculaire d'un vaisseau dans un corps humain ou animal, la gaine étant apte à être montée par étirement sur le ballonnet pour exercer une force de compression sur le ballonnet pour contracter radialement le ballonnet à partir de sa configuration étendue vers sa configuration contractée, la gaine comprenant :
au moins deux outils de traitement espacés entre eux (42, 50), la gaine étant **caractérisée en ce que** lesdits au moins deux outils de traitement espacés entre eux (42, 50) sont montés dans la gaine de façon à s'étendre radialement vers l'extérieur à partir du ballonnet, et **en ce que**, dans la configuration contractée du ballonnet, les outils sont protégés dans la gaine, et dans sa configuration étendue, l'épaisseur de la gaine diminue pour exposer les outils à un contact avec la zone cible de la paroi vasculaire.

13. Gaine selon la revendication 12, dans laquelle les outils de traitement sont des aiguilles (42).

14. Gaine selon la revendication 13, comprenant en outre :
une gaine intérieure (34) comprenant une surface extérieure (40) sur laquelle sont prévus une pluralité de réservoirs (38) pour stocker un composé thérapeutique ;
une gaine extérieure (36) disposée sur la gaine intérieure (34) ;
dans laquelle chacune des aiguilles (42) comprend une partie de base (46) et une partie d'injecteur (44), et dans laquelle chaque partie de base est disposée sur un réservoir sur la surface extérieure de la gaine intérieure, et dans laquelle chaque partie d'injecteur s'étend radialement vers l'extérieur à partir de la gaine intérieure et est reçue dans des trous coopératifs (39) définis dans la gaine extérieure.

15. Gaine selon la revendication 12, dans laquelle les outils de traitement sont des outils de coupe (50).

16. Gaine selon la revendication 13, comprenant au moins une protubérance (51) sur sa surface extérieure, dans laquelle, dans la configuration contractée du ballonnet, chaque protubérance s'étend radialement vers l'extérieur de la surface externe de la gaine plus loin que chaque outil de coupe.

17. Gaine selon la revendication 16, dans laquelle chaque protubérance est escamotable.

18. Gaine selon la revendication 16 ou 17, dans laquelle chaque protubérance comporte une poche creuse interne,
dans laquelle, dans la configuration étendue du ballonnet, la déformation de la gaine fait que la poche s'aplatit, réduisant ainsi la dimension de la protubérance dans la direction radiale pour exposer chaque outil de coupe.
